(19) Europäisches Patentamt
European Patent Office
Office européen des brevets

(11) Veröffentlichungsnummer: **0 022 751**
**B1**

# (12) EUROPÄISCHE PATENTSCHRIFT

(45) Veröffentlichungstag der Patentschrift: **18.05.83**

(51) Int. Cl.$^3$: **C 07 C 87/36,** C 07 C 85/06

(21) Anmeldenummer: **80810223.0**

(22) Anmeldetag: **07.07.80**

(54) **Verfahren zur Herstellung von 2,6-Dialkylcyclohexylaminen.**

(30) Priorität: **13.07.79 US 56164**

(43) Veröffentlichungstag der Anmeldung: **21.01.81 Patentblatt 81/3**

(45) Bekanntmachung des Hinweises auf die Patenterteilung: **18.05.83 Patentblatt 83/20**

(84) Benannte Vertragsstaaten: **AT BE CH DE FR GB IT LI NL SE**

(56) Entgegenhaltungen:
**DE-B-1 276 032**
**US-A-3 931 298**

(73) Patentinhaber: **CIBA-GEIGY AG, Patentabteilung Postfach, CH-4002 Basel (CH)**

(72) Erfinder: **Blackwell III, Joseph T., 4001 St. Patrick Drive, Greensboro, N.C. 27409 (US)**
Erfinder: **Grace, Henry C., 424 Ranchwood Drive, Baton Rouge Louisiana 70815 (US)**
Erfinder: **Nabors, James B., 526 Glendwild Drive, Baton Rouge La. 70815 (US)**
Erfinder: **Petree, Harris E., 1040, Kingsridge Road, Kernersville, N.C. 27284 (US)**

**EP 0 022 751 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

Verfahren zur Herstellung von 2,6-Dialkylcyclohexylaminen

Die vorliegende Erfindung betrifft ein Verfahren zur Herstellung von 2,6-Dialkylcyclohexylaminen der Formel I

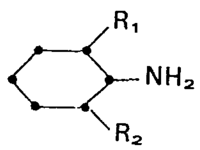

(I)

in welcher $R_1$ und $R_2$ unabhängig voneinander Methyl oder Äthyl bedeuten.

Die 2,6-Dialkylcyclohexylamine sind wertvolle Zwischenprodukte für die Herstellung von Herbiziden, Fungiziden, Pflanzenregulatoren und anderen biologisch aktiven Stoffen. Besonders wertvoll in bezug auf die Wirkung der daraus herstellbaren Endprodukte sind 2,6-Dimethylcyclohexylamin und 2-Äthyl-6-methylcyclohexylamin.

Es ist bekannt, Cyclohexylamine durch Hydrierung von entsprechenden Anilinen oder Nitrobenzolen oder durch Aminierung von entsprechenden Cyclohexanolen oder Cyclohexanonen herzustellen. Die Durchführung dieser Verfahren in technischem Massstab stösst insofern auf Schwierigkeiten, als die 2,6-Dialkylderivate der vorgenannten Ausgangsmaterialien nich gut zugänglich sind.

Es ist auch bekannt, Phenole in Gegenwart von Katalysatoren und in Gegenwart oder Abwesenheit von Wasserstoff mit Ammoniak zu den entsprechenden Anilinen umzusetzen.

In der US-A Nr. 3931298 ist die Umsetzung von 2,6-Dimethylphenol mit Ammoniak in Gegenwart von Wasserstoff, einem Wasserstoff übertragenden Katalysator und einem Cyclohexanon als Cokatalysator zu 2,6-Dimethylanilin beschrieben. Nach dieser Methode wird 2,6-Dimethylanilin in Ausbeuten von 65-78% erhalten. 2,6-Dimethylcyclohexylamin wird bei Durchführung dieses Verfahrens nur in Spuren gebildet.

In der US-A Nr. 3272865 wird die Umsetzung von Phenolen mit Ammoniak in Gegenwart von Aluminiumsilikat, Aluminiumtitanat, Aluminiumzirkonat, Phosphorsäure oder Wolframoxid als Katalysator zu den entsprechenden Anilinen beschrieben. In diesem Patent sind weder 2,6-Dialkylphenol und 2,6-Dialkylaniline noch die Bildung irgendwelcher Cyclohexylamine erwähnt.

In der DE-B Nr. 1276032 wird ein Verfahren zur Herstellung von Cyclohexylamin beschrieben, das darin besteht, dass man Phenol bei einer Temperatur von 70-150° C und in Gegenwart von 0,02-5 Gew.% metallischem Rhodium als Katalysator mit Ammoniak und Wasserstoff umsetzt. Die Umsetzung von substituierten Phenolen, insbesondere 2,6-disubstituierten Phenolen, in denen die beiden ortho-Substituenten die Hydroxygruppe sterisch abschirmen, wird nicht erwähnt.

Der vorliegenden Erfindung liegt die Aufgabe zugrunde, ein Verfahren bereitzustellen, nach dem 2,6-Dialkylcyclohexylamine der Formel I in industriellem Massstab aus leicht zugänglichen Ausgangsmaterialien hergestellt werden können.

Gemäss vorliegender Erfindung wird vorgeschlagen, die 2,6-Dialkylcyclohexylamine der Formel I durch Umsetzung eines 2,6-Dialkylphenols der Formel II

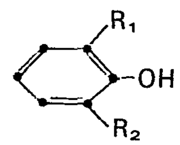

(II)

in welcher $R_1$ und $R_2$ die oben angegebene Bedeutung haben, mit Ammoniak in Gegenwart von Wasserstoff und einem Metall der Gruppe VIII des periodischen Systems in der Weise herzustellen, dass man die Umsetzung des 2,6-Dialkylphenols der Formel II bei einer Temperatur von 150-250° C, einem Druck von 7-21 bar und einem Molverhältnis von 2,6-Dialkylphenol der Formel II zu Ammoniak und Wasserstoff von 1:5-20:5-20 durchführt.

Das Verfahren kann diskontinuierlich und kontinuierlich durchgeführt werden. Es ist insbesondere für die kontinuierliche Durchführung geeignet.

Die erfindungsgemäss bevorzugt zu verwendenden Katalysatoren sind diejenigen Elemente der Gruppe VIII des periodischen Systems, welche eine ausgeprägte wasserstoffübertragende Wirkung haben. Die Katalysatoren werden vorzugsweise auf einem inerten Träger, wie Tonerde und Kohle, verwendet. Gute Resultate werden mit Platin oder Palladium auf Tonerde oder Kohle erhalten. Bevorzugt werden 0,1 bis 10%ige Platin- oder Palladiumkatalysatoren auf Kohle oder Tonerdegranulat als Träger verwendet.

Bei der diskontinuierlichen Durchführung des Verfahrens ist die Menge des Katalysators nicht kritisch und kann über einen weiten Bereich variieren. Gewöhnlich werden 0,5 bis 5 Gew.% Katalysator bezogen auf die Menge des eingesetzten 2,6-Dialkylphenols der Formel II verwendet. Bei der kontinuierlichen Durchführung des Verfahrens liegt das Verhältnis von Gewicht des Katalysators (W) zum Gewicht des pro Stunde über den Katalysator geleiteten 2,6-Dialkylphenols der Formel II (F) zweckmässig zwischen 0,05 und 10. Dieses Verhältnis (W/F) wird entsprechend der Aktivität des Katalysators verändert. Je aktiver die Katalysatoren sind, desto niedriger ist das W/F-Verhältnis. Falls notwendig, können die niedrigen W/F-Verhältnisse durch Zumischung von Glasperlen zum Katalysator erreicht werden.

Innerhalb des Temperaturbereichs von 150-250° C ist der Bereich von 175-225° C bevorzugt und innerhalb des Druckbereichs von 7-21 bar ist der Bereich von 12,5-16 bar bevorzugt.

Das Molarverhältnis von 2,6-Dialkylphenol der Formel II zu Ammoniak und Wasserstoff kann über einen weiten Bereich variieren. Wesentlich ist, dass Wasserstoff und Ammoniak im Überschuss

angewendet werden. In der Regel werden wenigstens 5 mol Ammoniak und 5 mol Wasserstoff pro Mol 2,6-Dialkylphenol der Formel II eingesetzt. In der Praxis beträgt das Molarverhältnis von 2,6-Dimethylphenol zu Ammoniak und Wasserstoff gewöhnlich 1:5-20:5-20. Vorzugsweise wird ein Molverhältnis von 2,6-Dimethylphenol zu Ammoniak und Wasserstoff von 1:10-15:10-15 angewendet.

Das erfindungsgemässe Verfahren wird vorzugsweise in einem röhrenförmigen Reaktor durchgeführt, in dem sich eine Katalysatorschüttung befindet und der mit entsprechenden Einlässen und Auslässen für Ausgangsmaterialien und Endprodukt versehen ist. Besonders geeignet ist ein senkrecht angeordneter Röhrenreaktor mit einer Kontaktschüttung im mittleren Teil und mit Einlässen für Ammoniak, Wasserstoff und 2,6-Dimethylanilin am Reaktorkopf und einem Sammelgefäss für das Endprodukt am Reaktorboden.

Die Reaktionszone wird mit einem 0,1-10%igen Palladium- oder Platinkatalysator auf Kohle oder Tonerdegranulat als Träger gefüllt. Der Katalysator wird in einem langsamen Strom von Ammoniak und Wasserstoff bei einer Temperatur im Bereich von 250-300° C aktiviert. Dann wird das 2,6-Dialkylphenol bei einer Temperatur von 175-225° C und einem Druck von 12,5 bis 16 bar über den aktivierten Katalysator geleitet, wobei das Molarverhältnis von 2,6-Dimethylphenol zu Ammoniak und Wasserstoff 1:10-15:10-15 beträgt. Das Produkt wird im Sammelgefäss am Boden des Reaktors gesammelt und in bekannter Weise, z.B. durch fraktionierte Destillation, gereinigt.

Mit den bevorzugten Katalysatoren und bei Einhaltung der bevorzugten Reaktionsbedingungen wird nur sehr wenig nicht umgesetztes 2,6-Dialkylphenol erhalten und nur sehr wenig Isomerisierung — d.h. Wanderung von Methylgruppen am carbocyclischen Ring — beobachtet. Sorgfältige Kontrolle von Temperatur, Druck und des W/F-Verhältnisses innerhalb der bevorzugten Bereiche gewährleistet guten Umsatz und gute Selektivität der Reaktion.

Aufgrund theoretischer Überlegungen, die durch die Isolierung von Zwischenprodukten und teilweise umgesetzten Materialien gestützt sind, liegt dem erfindungsgemässen Verfahren wahrscheinlich eine zweistufige Reaktion zugrunde, die durch das folgende Reaktionsschema beschrieben werden kann:

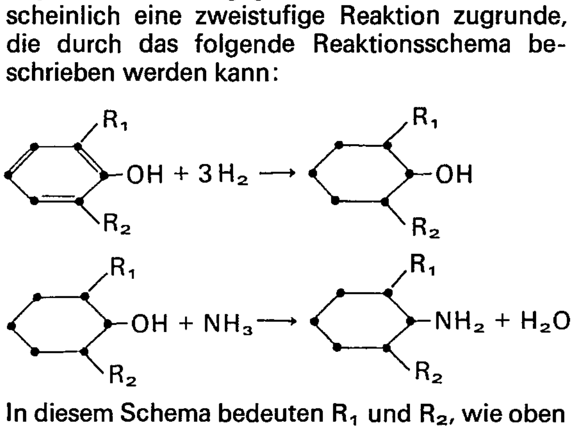

In diesem Schema bedeuten $R_1$ und $R_2$, wie oben angegeben, unabhängig voneinander Methyl oder Äthyl.

Wenn man in Betracht zieht, dass beide Reaktionen exotherm sind, wenn $R_1$ und $R_2$ je Methyl bedeuten, kann die Zusammensetzung des bei Durchführung des Verfahrens in einem kontinuierlichen Reaktor erhaltenen Produkts durch die angewandten Reaktionsbedingungen beeinflusst werden. 2,6-Dimethylphenol, 2,6-Dimethylcyclohexanol und 2,6-Dimethylcyclohexylamin werden unter den bevorzugten Bedingungen erhalten. Bei der Durchführung des Verfahrens unter milden Reaktionsbedingungen sind auch gerige Mengen an 2,6-Dimethylcyclohexanon beobachtet worden, die auf unvollständige Hydrierung zurückzuführen sind. Ebenso wurden geringe Mengen von nicht umgesetzten Ausgangsmaterial und etwas 2,6-Dimethylanilin beobachtet.

Gemäss einer bevorzugten Ausführungsform des erfindungsgemässen Verfahrens wird 2,6-Dimethylphenol bei einem Druck von 12,5-16 bar und einer Temperatur von 175-225° C über einen 0,1 bis 10%igen Platin- oder Palladiumkatalysator auf Kohle oder Tonerde als Träger geleitet, wobei das Molverhältnis von 2,6-Dimethylphenol zu Ammoniak und Wasserstoff 1:10-15:10-15 und das W/F-Verhältnis 2-6 beträgt.

Durch sorgfältige Abstimmung der wesentlichen Verfahrensparameter, wie Katalysator, Temperatur, Druck, W/F-Verhältnis und Mol-Verhältnis von 2,6-Dimethylphenol zu Ammoniak und Wasserstoff, kann ein kontinuierlicher Umsatz von mehr als 99% bezogen auf 2,6-Dimethylphenol und eine Selektivität in bezug auf die Bildung von 2,6-Dimethylcyclohexylamin von 80-97% erreicht werden. Diese Ergebnisse können beispielsweise durch Gas/Flüssig/Chromatographie (GLC) bestimmt werden.

Die folgenden Beispiele illustrieren bevorzugte Ausführungsformen des erfindungsgemässen Verfahrens. Durch diese Beispiele wird nicht beabsichtigt, die Erfindung in irgend einer Weise zu begrenzen. Die Beispiele sollen neben den konkret beschriebenen Ausführungsformen auch äquivalente Ausführungsformen mitumfassen. Die in den Beispielen beschriebenen Versuche wurden in einem senkrecht angeordneten röhrenförmigen Reaktor durchgeführt, in dessen mittleren Abschnitt sich die Katalysatorschüttung befand, und der am oberen Ende mit separaten Zuleitungen für Phenol, Ammoniak und Wasserstoff versehen war. Das am unteren Ende des Reaktors austretende Produkt wird in einer Vorlage gesammelt. Der Reaktor ist ausserdem mit einer Heizvorrichtung, geeigneten Temperatur- und Druckfühlern und Vorrichtungen zur Probenahme ausgestattet. Der Katalysator wird nach Einbringung in den Reaktor durch Erhitzen in einem Strom von Wasserstoff und Ammoniak etwa bis zu 3 h aktiviert. Wenn vom Hersteller nicht anders empfohlen, wird die Aktivierung bei 250° C während 2 h durchgeführt.

*Beispiel 1:*

Der Reaktor wird mit einem 0,5% Platin auf Kohlekatalysator beschickt. Der Katalysator wird bei 250° C in einem langsamen Strom von Wasserstoff und Ammoniak 2 h aktiviert. Dann wird bei einer

Temperatur von 225° C und einem Druck von 12,5 bar 2,6-Dimethylphenol, Ammoniak und Wasserstoff im Molverhältnis von 1:10:15 über den Katalysator geleitet, wobei das W/F-Verhältnis 5,9 beträgt. Nach GLC-Analyse beträgt der Umsatz 99,9% und die Selektivität 79%.

*Beispiel 2:*

Der Reaktor wird mit einem 0,5% Palladium auf Kohlekatalysator beschickt. Der Katalysator wird, wie in Beispiel 1 beschrieben, 2 h in einem langsamen Strom von Wasserstoff und Ammoniak aktiviert. Dann wird 2,6-Dimethylphenol mit einem W/F-Verhältnis von 2,95 über den aktivierten Katalysator geleitet. Das Molverhältnis von 2,6-Dimethylphenol zu Ammoniak und Wasserstoff beträgt 1:15:15. Durch GLC-Analyse des in der Vorlage erhaltenen Produkts wird ein Umsatz von 99,9% bezogen auf 2,6-Dimethylphenol und eine Selektivität von 97% bezogen auf die Bildung von 2,6-Dimethylcyclohexylamin bestimmt.

*Beispiel 3:*

Der Reaktor wird mit einem 1%igen Palladium auf Tonerdekatalysator beschickt. Die Aktivierung des Katalysators erfolgt wie in Beispiel 1 beschrieben. Dann wird bei 200° C und einem Druck von 16 bar 2,6-Dimethylphenol über den aktivierten Katalysator geleitet, wobei das W/F-Verhältnis 6,5 beträgt. Das Molverhältnis von 2,6-Dimethylphenol zu Ammoniak und Wasserstoff beträgt 1:15:15. Nach 29stündigem Betrieb des Reaktors wird durch GLC-Analyse des erhaltenen Produkts ein Umsatz von 97,48% bezogen auf 2,6-Dimethylphenol und eine Selektivität von 94,3% bezogen auf die Bildung 2,6-Dimethylcyclohexylamin bestimmt. Als weitere Produkte wurden 0,79% 2,6-Dimethylanilin, 1,34% 2,6-Dimethylcyclohexanol und 0,15% Dimethylcyclohexanon erhalten.

*Beispiel 4:*

Der Reaktor wird mit einem 0,5% Palladium auf Kohlekatalysator beschickt. Der Katalysator wird, wie in Beispiel 1 beschrieben, aktiviert. Dann wird bei 200°C und einem Druck von 16 bar 2-Äthyl-6-methylphenol mit einem W/F-Verhältnis von 3,5 über den Katalysator geleitet. Das Molverhältnis von 2-Äthyl-6-methylphenol zu Ammoniak und Wasserstoff beträgt 1:15:15. Gemäss GLC-Analyse des in der Vorlage erhaltenen Produkts wird ein Umsatz von 99,7% bezogen auf 2-Äthyl-6-methylphenol und eine Selektivität von 96,5% bezogen auf die Bildung von 2-Äthyl-6-methylcyclohexylamin erreicht.

## Patentansprüche

1. Verfahren zur Herstellung von 2,6-Dialkylcyclohexylaminen der Formel I

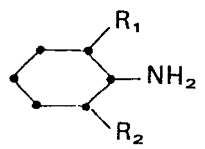

(I)

in welcher $R_1$ und $R_2$ unabhängig voneinander Methyl oder Äthyl bedeuten, durch Umsetzung eines 2,6-Dialkylphenols der Formel II

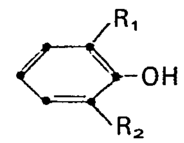

(II)

in welcher $R_1$ und $R_2$ die oben angegebene Bedeutung haben, mit Ammoniak in Gegenwart von Wasserstoff und einem Metall der Gruppe VIII des periodischen Systems, dadurch gekennzeichnet, dass man die Umsetzung des 2,6-Dialkylphenols der Formel II bei einer Temperatur von 150-250° C, einem Druck von 7-21 bar und einem Molverhältnis von 2,6-Dialkylphenol der Formel II zu Ammoniak und Wasserstoff von 1:5-20:5-20 durchführt.

2. Verfahren nach Anspruch 1, dadurch gekennzeichnet, dass man als Wasserstoffübertragungskatalysator Platin oder Palladium verwendet.

3. Verfahren nach Anspruch 1, dadurch gekennzeichnet, dass man als 2,6-Dialkylphenol der Formel II 2,6-Dimethylphenol oder 2-Äthyl-6-methylphenol verwendet.

4. Verfahren nach Anspruch 3, dadurch gekennzeichnet, dass man als 2,6-Dialkylphenol der Formel II 2,6-Dimethylphenol verwendet.

5. Verfahren nach Anspruch 1, dadurch gekennzeichnet, dass das Molverhältnis von 2,6-Dialkylphenol der Formel II zu Ammoniak und Wasserstoff 1:10-15:10-15 beträgt.

6. Verfahren nach Anspruch 1, dadurch gekennzeichnet, dass das Verhältnis des Gewichts des Katalysators zum Gewicht des pro Stunde über den Katalysator geleiteten 2,6-Dialkylphenols der Formel II (W/F) im Bereich von 0,05 bis 10 liegt.

7. Verfahren nach Anspruch 1, dadurch gekennzeichnet, dass man 2,6-Dimethylphenol bei einer Temperatur von 175-225° C und einem Druck von 12,5 bis 16 bar über einen 0,1-10%igen Platin- oder Palladiumkatalysator auf Kohle oder Tonerde als Träger leitet, wobei das Molverhältnis von 2,6-Dimethylphenol der Formel II zu Ammoniak und Wasserstoff 1:10-15:10-15 und das W/F-Verhältnis 2-6 beträgt.

## Claims

1. A process for the preparation of a 2,6-dialkylcyclohexylamine of formula I:

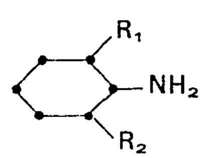

(I)

wherein $R_1$ and $R_2$ are each independently methyl or ethyl, by reaching a 2,6-dialkylphenol of formula II:

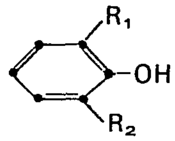

(II)

wherein $R_1$ and $R_2$ have the meaning given above, with ammonia, in the presence of hydrogen and a metal of group VIII of the Periodic Table, which process is carried out at a temperature within the range from 150 to 250° C and a pressure within the range from 7 to 21 bar, the molar ratio of 2,6-dialkylphenol of formula II to ammonia and hydrogen being from 1/5-20/5-20.

2. A process according to claim 1, wherein platinum or palladium is used as hydrogen transfer catalyst.

3. A process according to claim 1, wherein 2,6-dimethylphenol or 2-ethyl-6-methylphenol is used as 2,6-dialkylphenol of the formula II.

4. A process according to claim 3, wherein 2,6-dimethylphenol is used as 2,6-dialkylphenol of the formula II.

5. A process according to claim 1, wherein the molar ratio of the 2,6-dialkylphenol of formula II to ammonia and to hydrogen is 1/10-15/10-15.

6. A process according to claim 1, wherein the ratio of the weight of the catalyst to the weight of the 2,6-dialkylphenol of formula II passed per hour over the catalyst (W/F) is in the range from 0.05 to 10.

7. A process according to claim 1, wherein 2,6-dimethylphenol is passed in contact with the catalyst consisting of 0.1 to 10% of platinum or palladium on a carbon or alumina support, at a pressure in the range from 12.5 to 16 bar and a temperature in the range from 175° to 225° C, the molar ratio of 2,6-dimethylphenol of the formula II to ammonia and hydrogen being 1/10-15/10-15 and the W/F ratio being in the range from 2 to 6.

## Revendications

1. Procédé de préparation de 2,6-dialcoyl-cyclohexylamines de formule I:

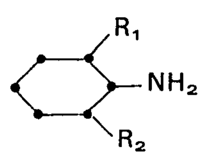

(I)

où $R_1$ et $R_2$ représentent indépendamment l'un de l'autre un méthyle ou un éthyle,
par réaction d'un 2,6-dialcoylphénol de formule II:

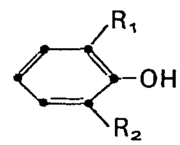

(II)

où $R_1$ et $R_2$ ont la signification donnée ci-dessus, avec de l'ammoniac en présence d'hydrogène et d'un métal du groupe VIII de la classification périodique des éléments, caractérisé en ce qu'on conduit la réaction du 2,6-dialcoylphénol de formule II à une température de 150 à 250° C, une pression de 7 à 21 bar et un rapport molaire du 2,6-dialcoylphénol de formule II à l'ammoniac et à l'hydrogène de 1/5-20/5-20.

2. Procédé selon la revendication 1, caractérisé en ce qu'on utilise comme catalyseur de transfert d'hydrogène le platine ou le palladium.

3. Procédé selon la revendication 1, caractérisé en ce qu'on utilise comme 2,6-dialcoylphénol de formule II le 2,6-diméthylphénol ou le 2-éthyl-6-méthylphénol.

4. Procédé selon la revendication 3, caractérisé en ce qu'on utilise comme 2,6-dialcoylphénol de formule II le 2,6-diméthylphénol.

5. Procédé selon la revendication 1, caractérisé en ce que le rapport molaire du 2,6-dialcoylphénol de formule II à l'ammoniac et à l'hydrogène s'élève à 1/10-15/10-15.

6. Procédé selon la revendication 1, caractérisé en ce que le rapport du poids du catalyseur au poids du 2,6-dialcoylphénol de formule II (W/F) que l'on fait passer par heure sur le catalyseur se situe dans un intervalle allant de 0,05 à 10.

7. Procédé selon la revendication 1, caractérisé en ce qu'on fait passer du 2,6-diméthylphénol à une température de 175 à 225° C et une pression de 12,5 à 16 bar sur un catalyseur au platine ou au palladium à 0,1 à 10% sur charbon ou argile comme support, le rapport molaire du 2,6-diméthylphénol de formule II à l'ammoniac et à l'hydrogène s'élevant à 1/10-15/10-15 et le rapport W/F à 2 à 6.